(19) Europäisches Patentamt / European Patent Office / Office européen des brevets

(11) **EP 4 365 282 B1**

(12) **EUROPEAN PATENT SPECIFICATION**

(45) Date of publication and mention
of the grant of the patent:
**07.05.2025 Bulletin 2025/19**

(21) Application number: **23195021.3**

(22) Date of filing: **04.09.2023**

(51) International Patent Classification (IPC):
**C12N 5/0783** (2010.01)   **C12N 5/10** (2006.01)
**A61K 39/00** (2006.01)   **A61K 35/17** (2025.01)
**A61P 35/00** (2006.01)

(52) Cooperative Patent Classification (CPC):
**C12N 5/0636; A61K 40/11; A61K 40/31;
A61P 35/00;** C07K 2319/03; C12N 2501/2302;
C12N 2501/51; C12N 2501/515; C12N 2510/00;
C12N 2531/00; C12N 2740/15043

(54) **METHOD FOR PRODUCING HUMAN CHIMERIC ANTIGEN RECEPTOR T-CELL POPULATION ENRICHED WITH STEM CELL-LIKE MEMORY T-CELLS**

VERFAHREN ZUR HERSTELLUNG EINER MIT STAMMZELLENÄHNLICHEN T-GEDÄCHTNISZELLEN ANGEREICHERTEN POPULATION MENSCHLICHER CHIMÄRER ANTIGENREZEPTOREN

PROCÉDÉ DE PRODUCTION D'UNE POPULATION DE LYMPHOCYTES T RÉCEPTEURS D'ANTIGÈNES CHIMÉRIQUES HUMAINS ENRICHIE EN LYMPHOCYTES T MÉMOIRES DE TYPE CELLULES SOUCHES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **03.11.2022 TW 111142071**

(43) Date of publication of application:
**08.05.2024 Bulletin 2024/19**

(73) Proprietor: **Pell Bio-Med Technology Co., Ltd.
Taipei City (TW)**

(72) Inventors:
• **LIN, Chien-Ting**
  **Taipei City (TW)**
• **LIN, Chen-Lung**
  **Taipei City (TW)**
• **LAI, Yi-Hui**
  **Taipei City (TW)**
• **LIN, Xuan-Hui**
  **Taipei City (TW)**
• **TUNG, Tzu-Hsun**
  **Taipei City (TW)**

(74) Representative: **Cabinet Chaillot
16/20, avenue de l'Agent Sarre
B.P. 74
92703 Colombes Cedex (FR)**

(56) References cited:
• **BLAESCHKE F ET AL: "Defined Central Memory and Stem Memory T Cell Phenotype of CD4 and CD8 CAR T Cells for the Treatment of CD19+ Acute Lymphoblastic Leukemia in an Automated Closed System", BLOOD, vol. 128, no. 22, 4558, 2 December 2016 (2016-12-02), 58th ASH Annual Meeting; San Diego, CA, USA; 3-6 December 2016, XP093128186, ISSN: 0006-4971, DOI: 10.1182/blood.V128.22.4558.4558**
• **ROUSSEL X ET AL: "Validation of an IL-1Rap CART-cells preclinical "GMP-like" production process before an upcoming first in human phase I clinical trial", HEMASPHERE, vol. 5, no. S2, EP720, 1 June 2021 (2021-06-01), 26th Congress of the European Hematology Association; virtual; 9-17 June 2021, pages 329 - 330, XP093128177, ISSN: 2572-9241, DOI: 10.1097/HS9.0000000000000566**

- **WANG S ET AL: "PI3K [delta] /[gamma] Inhibition Enhances the Expansion and Anti-Tumor Cytotoxicity of CART Cells for CLL Patients", BLOOD, vol. 138, no. Supplement 1, 5 November 2021 (2021-11-05), 63rd ASH Annual Meeting; Atlanta, GA, USA; 11-14 December 2021, pages 4795 - 4796, XP093128162, ISSN: 0006-4971, DOI: 10.1182/blood-2021-152163**
- **KAGOYA Y ET AL: "Transient stimulation expands superior antitumor T cells for adoptive therapy", JCI INSIGHT, vol. 2, no. 2, E89580, 26 January 2017 (2017-01-26), XP093071883, DOI: 10.1172/jci.insight.89580**
- **ARCANGELI S ET AL: "Next-Generation Manufacturing Protocols Enriching TSCM CAR T Cells Can Overcome Disease-Specific T Cell Defects in Cancer Patients", FRONTIERS IN IMMUNOLOGY, vol. 11, 1217, 19 June 2020 (2020-06-19), XP093082857, DOI: 10.3389/fimmu.2020.01217**
- **SABATINO M ET AL: "Generation of clinical-grade CD19-specific CAR-modified CD8+ memory stem cells for the treatment of human B-cell malignancies", BLOOD, vol. 128, no. 4, 28 July 2016 (2016-07-28), pages 519 - 528, XP055340306, ISSN: 0006-4971, DOI: 10.1182/blood-2015-11-683847**

**Description**

1. Field of the Invention

The present invention relates to a method for producing human chimeric antigen receptor T cells (CAR-T cells), and particularly to a method for producing human CAR-T cells enriched with stem cell-like memory T-cells (Tscm).

2. Description of the Prior Arts

CAR-T cell therapy uses gene editing technology to enable T cells to express receptors that can recognize surface antigens of tumor cells. Once modified T cells are infused back into the human body, the modified T cells can recognize and kill tumor cells.

However, this therapy also has side effects, the most severe adverse effect being cytokine release syndrome (CRS). CRS can be described by large amounts of cytokines released when CAR-T cells fight against tumor cells, which in turn results in excessive inflammation, microvascular leakage, and initiation of chain reactions that lead to coagulation. Moreover, it may lead to life threatening events of heart, lung, kidney failure or brain swelling. It is commonly believed that the abundance of Tscm in CAR-T cells can prolong the survival time of CAR-T cells in the human body, increase the therapeutic efficacy, and reduce the incidence and severity of CRS and neurological toxicity.

However, the proportion of Tscm cells in the peripheral blood of the human body is only about 1% to 5%. Conventional methods for producing CAR-T cells cannot effectively generate CART with high proportions and numbers of Tscm cells. Since the proportion of Tscm within conventionally prepared CAR-T is not high enough, such treatment not only runs a higher risk of causing severe CRS and neurotoxicity but has also been associated with reduced efficacy relating to a shorter *in vivo* lifespan. The production time required to generate *in vitro* CAR-T cells is also lengthy, ranging between 9 to 14 days, in order to produce sufficient CAR-T cells for treatment.

To overcome the shortcomings of the existing production technology, the purpose of the present invention is to provide a method to produce sufficient CAR-T cells with a short production period, which is also rich in Tscm.

To achieve the aforementioned purpose, the present invention provides a method for producing human chimeric antigen receptor T (CAR-T) cells enriched with stem cell-like memory T-cells, comprising: step (1) providing human peripheral blood mononuclear cells (PBMCs); step (2) positively selecting human T cells from the human PBMCs by forming human T cell-magnetic bead complexes, through mixing anti-CD3 and anti-CD28 antibodies coated magnetic beads with the human PBMCs, which also contain B cells, Nature Killer (NK) cells and monocytes; step (3) culturing the human T cell-magnetic bead complexes for 16 hours to 20 hours for T cell activation to obtain activated human T cell-magnetic bead complexes; step (4) adding CAR-expressing lentivirus to the activated human T cell-magnetic bead complexes for 40 hours to 48 hours, for transduction of human T cells into CAR-T cells on the human T cell-magnetic bead complexes to obtain human CAR-T cell-magnetic bead complexes; step (5) obtaining human CAR-T cells (without magnetic beads) by dissociating the human CAR-T cells from the human CAR-T cell-magnetic bead complexes; step (6) further culturing the human CAR-T cells for 92 hours to 96 hours to obtain the human CAR-T cells enriched with Tscm.

Described within the current invention, by controlling the contact time between human T cells and magnetic beads; the human CAR-T cells produced through 7 days of culture exhibited the greatest expansion rate while also being enriched with Tscm. The present invention provides a method to create human CAR-T cells with greater immune function, than those prepared using existing technology, and is therefore more suitable for CAR-T immunotherapy.

According to the present invention, the human PBMCs in step (1) are either isolated from human peripheral blood or are from leukapheresis product, and such PBMCs include cells such as T cells, B cells, NK cells and monocytes.

According to the present invention, the source of human PBMC described herein can be from a healthy donor or cancer patient.

According to the present invention, the magnetic beads can activate human T cells. Preferably, the magnetic beads are Dynabeads™ Human T-Expander CD3/CD28 (Gibco, Cat. No. 11141D) or CTS™ (Cell Therapy Systems) Dynabeads™ CD3/CD28 (Gibco, CAT. No. 40203D).

Preferably, the number of the anti-CD3 and anti-CD28 antibodies coated magnetic beads in step (2) is 2 to 4 folds of the number of human T cells in the human PBMCs. More preferably, the number of the magnetic beads is 3 folds of the number of human T cells in the human PBMCs.

Preferably, the ratio of the total volume (in mL) of complete X-VIVO™ 15 medium to the culture area (in $cm^2$) of a culture flask in steps (3) and (4) is from 0.12 mL/$cm^2$ to 0.3 mL/$cm^2$. That is the culture medium has a volume to surface area ratio of 0.12 mL/$cm^2$ to 0.3 mL/$cm^2$ when filled in the culture flask. More preferably, the ratio of the total volume of complete X-VIVO™ 15 medium to the culture area of a culture flask in steps (3) and (4) is from 0.2 mL/$cm^2$ to 0.27 mL/$cm^2$. For example, when the culture area of a culture flask is 75 $cm^2$, the total volume of complete X-VIVO™ 15 medium applied is from 9 mL to 22.5 mL. More preferably, when the culture area of a culture flask is 75 $cm^2$, the complete X-VIVO™ 15 medium applied is between 15 mL and 20.25 mL.

Preferably, the number of the magnetic beads in step (2) is controlled to be 2 to 4 folds of the number of human T cells in the human PBMCs and the ratio of the total volume of the complete X-VIVO™ 15 medium to the culture area of a flask in steps (3) and (4) is controlled to be 0.12 mL/cm$^2$ to 0.3 mL/cm$^2$ simultaneously. In other words, by controlling the contact time of human T cells and magnetic beads, the ratio between the magnetic beads and human T cells in the step (2), and the ratio between the volume of the complete X-VIVO™ 15 medium and the culture area of a culture flask in the steps (3) and (4), simultaneously, T cells can be expanded to many folds in a short period of time, with a large population being Tscm.

Preferably, the human T cell-magnetic bead complexes in step (3) is cultured at 36.5°C to 37.5°C for 16 hours to 20 hours. For example, it can be for 16 hours to 19 hours or 17 hours to 19 hours. More preferably, the human T cell-magnetic bead complexes in step (3) is incubated in a humidified environment containing 5% $CO_2$ at 37°C for 18 hours.

Preferably, the lentivirus transduction in step (4) is conducted at 36.5°C to 37.5°C for 40 hours to 56 hours. More preferably, the lentivirus transfection in step (4) is conducted in a humidified environment containing 5% $CO_2$ at 37°C for 48 hours.

Preferably, in step (6), the human CAR-T cells are cultured at 36.5°C to 37.5°C for 92 hours to 96 hours. More preferably, in step (6), the human CAR-T cells are cultured in a humidified environment containing 5% $CO_2$ at 37°C for 96 hours.

Preferably, the culturing of the human T cell-magnetic bead complexes in step (3), the lentivirus transduction in step (4), and culturing of the human CAR-T cells in step (6) are performed by using a complete X-VIVO™ 15 (Longa, Cat. No. 04-418Q) medium containing IL-2, wherein the concentration of IL-2 in the complete X-VIVO™ 15 medium is 200 IU/mL.

According to the present invention, the complete X-VIVO™ 15 medium is X-VIVO™ 15 medium containing 200 IU/mL of recombinant human IL-2 (R&D Systems, Cat No. 202-IL-500).

Preferably, the lentivirus transduction in step (4) is the transduction of a gene comprising anti-CD19, anti-CD22, anti-B-cell maturation antigen (BCMA), or anti-mesothelin receptor sequences into human T cells.

Preferably, the number of human CAR-T cells in the human CAR-T cell population enriched with Tscm obtained in step (6) is more than 10-fold of the number of human T cells in the human T cell-magnetic bead complexes in step (2), *i.e.* the number of human T cells isolated using magnetic beads in step (2). That is, the number of human CAR-T cells obtained by culture expansion in step (6) is more than 10-fold of the number of human T cells in the human T cell-magnetic bead complexes in step (2). More preferably, the number of the human CAR-T cells enriched with Tscm obtained in step (6) is more than 20-fold of the number of human T cells in the human T cell-magnetic bead complexes in step (2). Even more preferably, the number of human CAR-T cells enriched with Tscm obtained in step (6) is more than 30-fold of the number of human T cells in the human T cell-magnetic bead complexes in step (2).

Preferably, the culturing of the human CAR-T in step (6) is performed using a medium mixture, wherein the medium mixture is a mixture comprising fresh complete X-VIVO™ 15 medium with conditioned medium of the human CAR-T cells obtained in step (5). That is, fresh complete X-VIVO™ 15 medium is added to the conditioned medium from culturing the human CAR-T cells, which underwent step (1) to step (5). Preferably, the volume ratio of the fresh complete X-VIVO™ 15 medium to the conditioned medium from the human CAR-T cells obtained in step (5) is from 1: 1 to 3: 1.

Preferably, of the Tscm-enriched human CAR-T cells enriched with Tscm obtained from step (6), the percentage of Tscm is higher than 55%. That is, of the total human CAR-T cells obtained following culture expansion in step (6) more than 55% are Tscm. More preferably, of the human CAR-T cells enriched with Tscm obtained from step (6), the percentage of the Tscm is higher than 60%. More preferably, of the human CAR-T cells enriched with Tscm obtained from step (6), the percentage of the Tscm is higher than 70%. Even more preferably, for the human CAR-T cells enriched with Tscm obtained from step (6), the percentage of the Tscm is higher than 75%.

Preferably, the present invention further comprises step (7) cryo-preserving the human CAR-T cells enriched with Tscm.

The advantages of the current invention are manufacturing procedures of CAR-Ts that induce substantial expansion of T cells in a short period of time and produce human CAR-T cells rich in Tscm, which can further differentiate, have a longer life-span, and less likely to lead to side effects such as CRS and neurotoxicity. Therefore, the human CAR-T cells produced by the method of the present invention are more suitable for CAR-T cell therapy.

## IN THE DRAWINGS

FIG. 1 shows the expansion folds of human CAR-T cells at day 7 compared to day 0, for Example and Comparative Example.

FIG. 2 shows the expansion folds of human T cells of Example on Day 7 and of Comparative Example on Day 9, compared to day 0.

FIG. 3 shows human T cell subpopulations of Example on Day 7 and of Comparative Example on Day 9.
FIG. 4A is the flow cytometry of human T cell subpopulations for Example on Day 7.
FIG. 4B is the flow cytometry of human T cell subpopulations for Comparative Example on Day 9.

**Example**

First, **step (1), human PBMCs were provided** on Day 0. Specifically, the whole blood of healthy human donor was collected and evenly mixed with an equal volume of 2% albumin/phosphate buffered saline buffer (Alb/PBS buffer), and a diluted whole blood sample was obtained. Human PBMCs were then isolated from the diluted whole blood sample using SepMate™-50 tubes containing inserts and by density gradient centrifugation. The detailed procedures are described as the following: 15 mL of Ficoll®-Paque Premium density gradient medium (GE Healthcare) was added to the SepMate™-50 tube by carefully pipetting it through the central hole of the SepMate™ insert. Then, the diluted whole blood sample was slowly added into the SepMate™-50 tube by pipetting it down the side of the tube. The SepMate™-50 tube was centrifuged at 1200 $\times$g for 20 minutes at room temperature using a centrifuge with the brake on. After centrifugation, the top layer, which was expected to contain human PBMCs, was poured into a new tube and then centrifuged at 300 $\times$g for 8 minutes at room temperature. The supernatant was then discarded, and the human PBMC cell pellet was resuspended with an appropriate volume of 2% Alb /PBS buffer to adjust the human PBMC concentration to 2 to $5\times10^7$ cells/mL.

The cell count was conducted by first sampling 7.5 $\mu$L of cell suspension and mixing it with 6 $\mu$L of PBS buffer as well as 1.5$\mu$L of the Acridine Orange/Propidium Iodide Stain by pipetting. Then, a LUNA-FL™ Automated Fluorescence Cell Counter was used to measure the density, viability, and size of the cell sample. The total cell number in the suspension was calculated by multiplying the viable cell density with the total cell suspension volume.

Moreover, prior to performing step (2), it was necessary to know the percentage of CD3+ T cells in the human PBMCs in order to calculate the number of magnetic beads required for step (2). To achieve this, 10 $\mu$L of the MACS 8-color immunophenotyping cocktail and 1 $\mu$L of the 7-AAD Staining Solutions were used to stain $1\times10^5$ of PBMCs, by co-incubation for 10 minutes in the dark at 4°C. 2 mL of 2% hAlb/Saline buffer was added to wash cells, which were then centrifuged at 350$\times$g for 5 minutes at room temperature. Following that, supernatants were discarded, and the cell pellet were gently tapped and resuspended with 200 $\mu$L of 2% Alb /Saline buffer. The cell subpopulations of PBMCs was analyzed using a Flow Cytometry (CytoFLEX), with the percentage of CD3+ T cells generally being around 40% to 70%.

The following formula was applied to calculate the number of cells presenting surface antigen CD3+ in human PBMCs, namely, the number of human T cells:

$$\text{Total PBMCs} \times \text{CD3}^+ \text{ T cell \%} = \text{number of CD3}^+ \text{ T cells}$$

Next, **step (2), antibody-coated magnetic beads were mixed with human PBMCs to positively select T cells from human PBMCs that also contain B cells, Nature Killer (NK) cells and monocytes, via forming human T cell-magnetic bead complexes, wherein the magnetic beads are coated with anti-CD3 and anti-CD28 antibodies.** Specifically, 2% hAlb/PBS buffer was added to the tube to resuspend the human PBMCs, adjusting the human T cell density to 2 to $5\times10^7$ cells/mL, while not exceeding a maximum of $2\times10^8$ total nucleated cells (PBMCs) per mL. Prior to mixing with PBMCs, magnetic beads coated with anti-CD3/CD28 antibodies (Dynabeads Human T-Expander CD3/CD28: Gibco, Cat. No. 11141D) were washed twice with 2%Alb/PBS buffer. The magnetic beads were added to the human PBMCs suspension to obtain a mixture of human PBMCs and magnetic beads, wherein the ratio between the number of magnetic beads to the number of human T cells was 3: 1. Human PBMCs and magnetic beads were mixed by rotating the tubes at 6 rpm for 30 minutes at room temperature. After mixing, the tube containing human PBMCs and magnetic beads was placed in a magnet for 1 to 2 minutes followed by disposing of the supernatant that contained unbound cells, such as B cells, NK cells, and monocytes in human PBMCs. Most human T cells that express CD3 will bind to magnetic beads by antigen-antibody interaction and form T cell-magnetic bead complexes, which will be obtained through this process.

Before step (3), the number of human T cells bound to magnetic beads was calculated. Complete X-VIVO™ 15 medium (X-VIVO™ 15 medium containing 200 IU/mL of recombinant human IL-2 (R&D Systems, Cat No. 202-IL-500)) was added at 5 times to the suspension volume of human T cell-magnetic bead complexes sample, to readjust the density of the human T cell-magnetic bead complexes. 7.5 $\mu$L of suspension comprising the human T cell-magnetic bead complex was sampled and mixed with 6 $\mu$L of PBS buffer and 1.5$\mu$L of the Acridine Orange/Propidium Iodide Stain. The number of human T cells bounded to the magnetic beads was then determined by an Automated Fluorescence Cell Counter.

**Step (3), human T cell-magnetic bead complexes were cultured for 16 to 20 hours for T cell activation and to obtain activated human T cell-magnetic bead complexes.** Specifically, the human T cell-magnetic bead complexes were cultured with appropriate volumes of complete X-VIVO™ 15 medium for a human T cell density of $1\times10^6$ cells /mL in T-flask(s), and incubated in a humidified environment containing 5% $CO_2$ at 37°C for 20 hours to obtain the activated human T cell-magnetic bead complexes.

Next, on Day 1 of the present Example, **step (4), CAR-expressing lentivirus was added to the culture of activated human T cell-magnetic bead complexes, for the transduction of human T cell into CAR-T cells on the cell-magnetic bead complexes.** Specifically, lentivirus was added to the culture of activated human T cell-magnetic bead complexes obtained from step (3) at multiplicity of infections (MOIs) of 0.5 to 3, through even mixing while using mild force and speed to prevent dissociation between human T cells and the magnetic beads. After 30 seconds of mixing, the culture was returned to incubation, in a humidified environment containing 5% $CO_2$ at 37°C, for another 48 hours, for lentivirus transduction so that human CAR-T cell-magnetic bead complexes were created.

On Day 3 of the present Example, **step (5), human CAR-T cells (without magnetic beads) were obtained from the human CAR-T cell-magnetic bead complexes via** dissociation. First, the total volume of the human CAR-T cell-magnetic bead complexes culture was measured. Then, 7.5 $\mu$L of the human CAR-T cell-magnetic bead complexes culture was sampled and mixed with 6 $\mu$L of PBS buffer and 1.5$\mu$L of the Acridine Orange/Propidium Iodide Stain. A LUNA-FL™ Automated Fluorescence Cell Counter was used to measure the density, viability, and size of the cell sample. The total number of human CAR-T cells (in the form of human CAR-T cell-magnetic bead complex) in the suspension was calculated by multiplying the viable cell density with the total volume of the human CAR-T cell-magnetic bead complexes culture. Tubes containing cell suspensions comprised of the human CAR-T cell-magnetic bead complexes were gently pipetted to dissociate the CAR-T cells and magnetic beads, then placed in a magnet for 1 to 2 minutes, to enable fixing and removing of the magnetic beads from the cell sample. This step was repeated three times, to ensure adequate removal of magnetic beads from the cell samples and for the collection of a cell suspension comprised of only human CAR-T cells.

Before step (6), the number and recovery rate of the human CAR-T cells from the CAR-T cell-magnetic bead complexes culture were calculated. Specifically, the total volume of the human CAR-T cell suspension was determined. 7.5 $\mu$L of suspension comprising human CAR-T cells was sampled and mixed with 6 $\mu$L of PBS buffer and 1.5 $\mu$L of the Acridine Orange/Propidium Iodide Stain. Then, a LUNA-FL™ Automated Fluorescence Cell Counter was used to measure the density, viability, and size of the cell sample. The total number of CAR-T cells in the suspension was calculated by multiplying the viable cell density with the total human CAR-T cell suspension volume. With this, the cell recovery rate (%) of the magnetic beading removal procedure was also determined.

Lastly, step (6), **human CAR-T cells were further cultured to obtain human CAR-T cells enriched with Tscm.** Specifically, fresh complete X-VIVO™ 15 medium was added to the original conditioned medium of human CAR-T cells, with human CAR-T cells in the suspension, to adjust the cell density to $7 \times 10^5$ cells/mL in T-flask(s). These T-flask(s) were incubated in a humidified environment containing 5% $CO_2$ at 37°C for a total of 96 hours (4 days). During this step, not only do the human CAR-T cells further expanded, their Tscm population is also enriched. The product obtained through this step is human CAR-T cells enriched with Tscm. Also, appropriate volumes of fresh complete X-VIVO™ 15 medium were added in addition, to the conditioned medium every 2 to 3 days, to maintain the CAR-T cell density at $7 \times 10^5$ cells/mL within the same flask. Whilst, when performing adjustments to the cell density by adding fresh complete X-VIVO™ 15 medium, a dilution to less than 3-fold dilution is preferred.

**Comparative Example**

The method of Comparative Example was similar to the Example, except for between steps (4) and step (6). For the Example, step (5) removal of the magnetic beads was conducted after 48 hours of lentiviral transduction as mentioned in step (4). While in Comparative Example, the magnetic beads were not removed until after 96 hours of lentivirus transduction. That is, the method of the Comparative Example was: **step (4') lentivirus was added to the culture of activated human T cell-magnetic bead complexes for 96 hours, for the transduction of human T cell into CAR-T cells on the cell-magnetic bead complexes,** performed on Day 1 of producing the Comparative Example. Additional medium was also supplied at 48 hours following the initiation of lentivirus transduction. Then, **step (5') magnetic beads were removed from the human CAR-T cell-magnetic bead complexes to obtain human CAR-T cells** on Day 5 of producing the Comparative Example. Finally, step (6') **the human CAR-T cells were cultured for 96 hours at 37°C to obtain expanded human CAR-T cells.**

Magnetic beads can activate, stimulate and expand T cells, so compared to the Example, T cells of the Comparative Example were actually activated by the magnetic beads for a longer period of time.

**Test Example 1. Determining the Expansion Fold of T Cells**

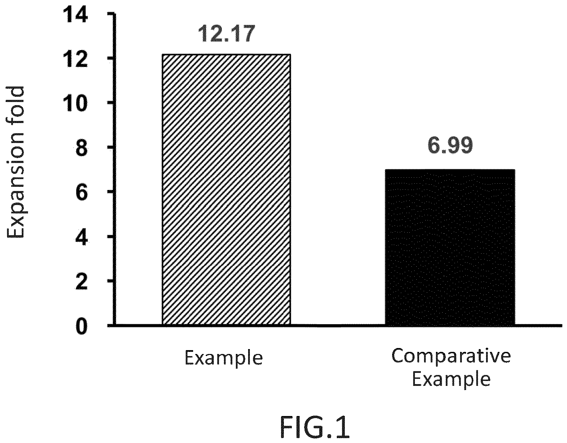

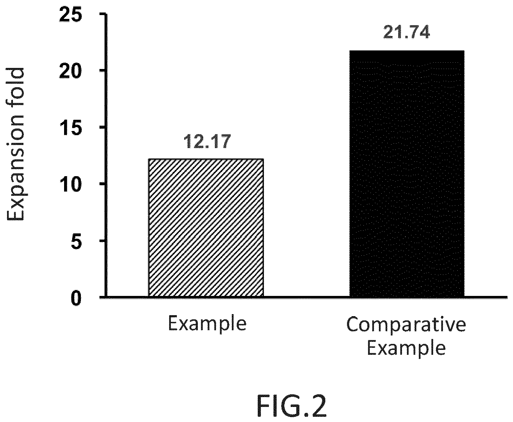

7.5 $\mu$L of cell suspension comprising expanded human CAR-T cells obtained from step (6) of Example and step (6') of Comparative Example were sampled and mixed with 6 $\mu$L of PBS buffer and 1.5 $\mu$L of the Acridine Orange/Propidium Iodide Stain, respectively. Then, a LUNA-FL™ Automated Fluorescence Cell Counter was used to measure the density, viability, and size of the cell sample. The total number of human CAR-T cells in Example and in Comparative Example were calculated by multiplying the viable cell density with the total cell suspension volumes and respectively compared with their total human T cells measured on Day 0. FIG. 1 shows the expansion fold results at Day 7 of culture for Example and Comparative Example. FIG. 2 shows the expansion fold results of the expanded human CAR-T cells obtained after 96 hours (4 day) of culture following magnetic beads removal, for Example (Day 7) and Comparative Example (Day 9). In addition, the total number of human CAR-T cells of the Example on the Day 0, Day 3, Day 6, and Day 7 and that of the Comparative Example on Day 0, Day 5, Day 6, Day 7, Day 8, Day 9 are listed in the following Table 1.

Table 1: Total numbers of human CAR-T cells of the Example and of the Comparative Example on different days of the producing procedure.

| Day \ Group | Day 0 | Day 3 | Day 5 | Day 6 | Day 7 | Day 8 | Day 9 |
|---|---|---|---|---|---|---|---|
| Example | $3.00 \times 10^6$ | $2.72 \times 10^6$ | | $3.09 \times 10^7$ | $3.65 \times 10^7$ | | |
| Comparative Example | $2.82 \times 10^6$ | | $9.97 \times 10^6$ | $9.79 \times 10^6$ | $1.97 \times 10^7$ | $2.97 \times 10^7$ | $6.13 \times 10^7$ |

According to the results in FIG.1, FIG.2 and Table 1, the total number of expanded human CAR-T cells obtained from Example on Day 7 shows 12.17 expansion folds compared to the total number of human T cells on Day 0. Although the total cell number obtained and the expansion fold of Comparative Example on Day 9 are higher than those of Example on Day 7, the total cell number of Comparative Example on Day 7 is $1.97 \times 10^7$, which is much lower than that of Example $(3.65 \times 10^7)$. Moreover, an expansion fold of 6.99 for the Comparative Example on Day 7, relative to Day 0, is only about half of that for the Example. Therefore, this proves that the present invention can make T cells grow more rapidly and expand excessively.

**Test Example 2. Characterization of Human T Cell Subpopulations**

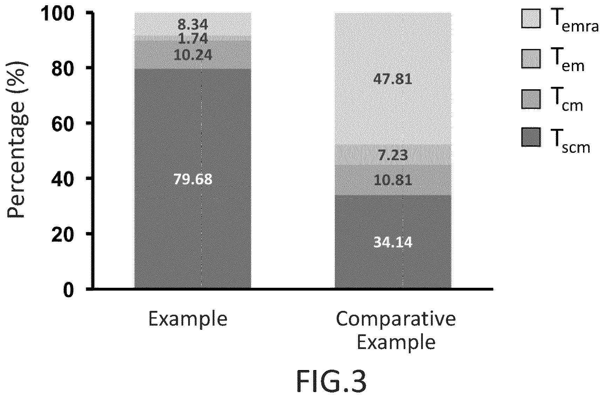

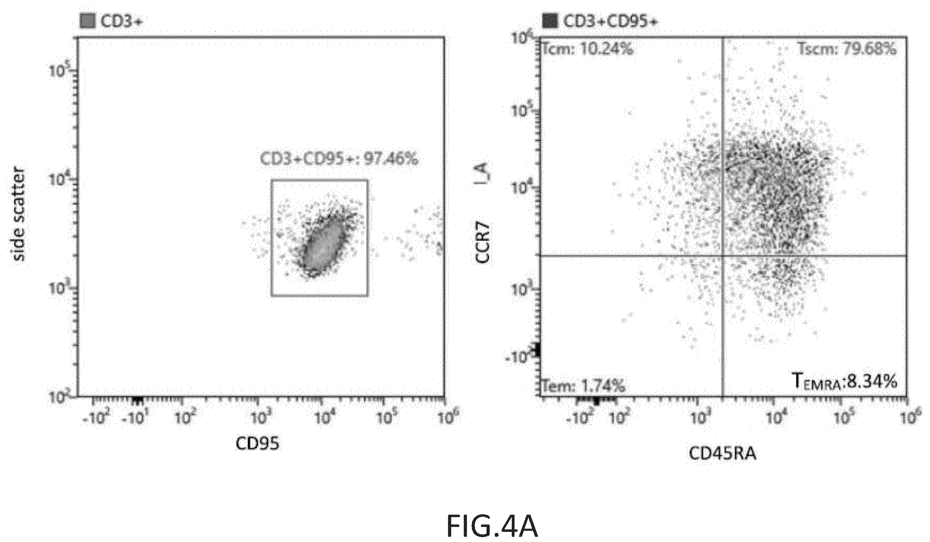

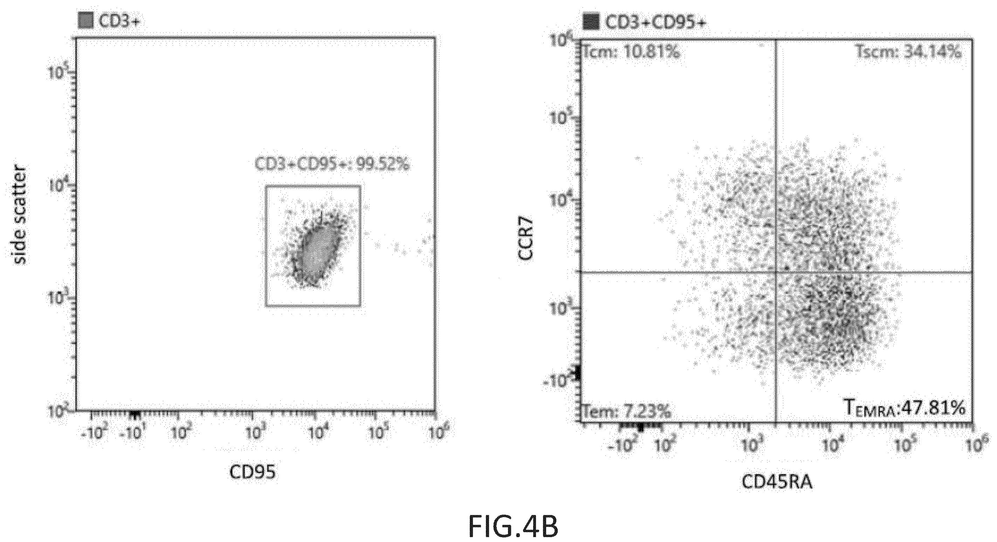

The expanded human CAR-T cells obtained from step (6) of Example and step (6') of Comparative Example were analyzed by flow cytometry after fluorescent labelling of surface markers of CD3, CD4, CD8, CD95, CD45RA, and CCR7 to characterize the human T cell subpopulations, including Tscm, central memory T cell ($T_{CM}$), effector memory T cell ($T_{EM}$), and terminally differentiated effector memory T cell ($T_{EMRA}$). The results are shown in FIGs 3, 4A, 4B and in Table 2.

Table 2: Percentage T cell subpopulations of expanded human CAR-T cells for Example on Day 7 and Comparative Example on Day 9.

| T cell subpopulations \ Day and Group | Tscm | $T_{CM}$ | $T_{EM}$ | $T_{EMRA}$ |
|---|---|---|---|---|
| Day 7 of Example | 79.68 | 10.24 | 1.74 | 8.34 |
| Day 9 of Comparative Example | 34.14 | 10.81 | 7.23 | 47.81 |

In the whole blood of a healthy donor, Tscm is typically 2% to 3% of the total T cell population (Gattinoni, Luca, et al. "A human memory T cell subset with stem cell-like properties." Nature medicine 17.10 (2011): 1290-1297). According to the results of Table 2, FIG. 3, FIG. 4A, and FIG. 4B, Tscm was 79.68% of the expanded human CAR-T cells in the Example, which is much higher than the proportion of Tscm of 34.14% in the Comparative Example. Besides, the total number of Tscm in Example is $2.91 \times 10^7$ ($3.65 \times 10^7 \times 79.68\% = 2.91 \times 10^7$), while the total number of Tscm in Comparative Example is $2.09 \times 10^7$ ($6.13 \times 10^7 \times 34.14\% = 2.09 \times 10^7$), which translates to a 39% difference in percentage Tscm between the Example and the Comparative Example. In addition, the proportion of $T_{EMRA}$ of the Example is 8.35%, which is significantly lower than that of the Comparative Example (47.81%). Conceptually, this means that the majority of T cells of the Example are most likely in a less differentiated state and not as senescent, so they are expected to retain the capability to differentiate further into $T_{CM}$ or $T_{EM}$, have longer lifespan than $T_{CM}$, $T_{EM}$, and $T_{EMRA}$, and also have self-renewal capacities. Consequently, the human CAR-T cells that are enriched with a Tscm population, such as the ones produced for the Example of the present invention, are more suitable for CAR-T cell therapy compared to those produced as the Comparative Example. Moreover, human CAR-T cells produced using the present invention can potentially reduce sides effects of CRS and neurotoxicity induced by CAR-T cell therapy.

To sum up, the method of the present invention can produce human CAR-T cells enriched with a Tscm population in a shorter period of time. The produced human CAR-T cells possess high proportions of Tscm, so they have greater capabilities to further differentiate, can last longer, and potentially reduce side effects of CRS and neurotoxicity induced by CAR-T cell therapy. Therefore, the human CAR-T cells produced by the method of the present invention are more suitable for the CAR-T cell therapy.

**Claims**

1. A method for producing human chimeric antigen receptor T (CAR-T) cells enriched with stem cell-like memory T-cells (Tscm), **characterized in that**, the method comprises:

    step (1) providing human peripheral blood mononuclear cells (PBMCs);
    step (2) positively selecting human T cells from the human PBMCs by forming human T cell-magnetic bead complexes, through mixing anti-CD3 and anti-CD28 antibodies coated magnetic beads with the human PBMCs;
    step (3) culturing the human T cell-magnetic bead complexes for 16 hours to 20 hours for T cell activation to obtain activated human T cell-magnetic bead complexes;
    step (4) adding CAR-expressing lentivirus to the activated human T cell-magnetic bead complexes for 40 hours to 48 hours, for transduction of human T cells into CAR-T cells on the human T cell-magnetic bead complexes to obtain human CAR-T cell-magnetic bead complexes;
    step (5) obtaining human CAR-T cells by dissociating the human CAR-T cells from the human CAR-T cell-magnetic bead complexes; and
    step (6) further culturing the human CAR-T cells for 92 hours to 96 hours to obtain the human CAR-T cells enriched with Tscm.

2. The method according to claim 1, wherein the human PBMCs in step (1) are collected by isolation from human whole blood or leukapheresis product.

3. The method according to claim 1 or 2, wherein the number of the anti-CD3 and anti-CD28 antibodies coated magnetic beads in step (2) is 2 to 4 folds of the number of human T cells in the human PBMCs.

4. The method according to any one of claims 1 to 3, wherein the human T cell-magnetic bead complexes in step (3) is cultured at 36.5°C to 37.5°C for 16 hours to 20 hours.

5. The method according to any one of claims 1 to 4, wherein the transduction in step (4) is conducted at 36.5°C to 37.5°C for 40 hours to 48 hours.

6. The method according to any one of claims 1 to 5, wherein the transduction in step (4) is transduction of a gene comprising anti-CD19, anti-CD22, anti-B cell maturation antigen (BCMA) or anti-mesothelin receptor sequences into human T cells.

7. The method according to any one of claims 1 to 6, wherein the human CAR-T cells are cultured at 36.5°C to 37.5°C for 92 hours to 96 hours in step (6).

8. The method according to any one of claims 1 to 7, wherein the culturing the human T cell-magnetic bead complexes in step (3), the transduction of human T cells into CAR-T cells on the human T cell-magnetic bead complexes in step (4), and the further culturing the human CAR-T cells in step (6) are performed in complete X-VIVO™ 15 medium.

9. The method according to any one of claims 1 to 8, wherein the number of the human CAR-T cells enriched with Tscm obtained in step (6) is more than 10-fold of the number of human T cells positively selected using magnetic beads in step (2).

10. The method according to any one of claims 1 to 9, wherein, the human CAR-T cells enriched with Tscm, which is obtained from step (6), exhibits a percentage of Tscm higher than 55%.

**Patentansprüche**

1.  - Verfahren zum Herstellen von humanen T-Zellen mit chimären Antigenrezeptoren (CAR-T-Zellen), die mit stammzellähnlichen T-Gedächtniszellen (Tscm) angereichert sind, **dadurch gekennzeichnet, dass** das Verfahren umfasst:

    Schritt (1), Bereitstellen von humanen mononukleären Zellen aus peripherem Blut (PBMCs);
    Schritt (2), positives Selektieren von humanen T-Zellen aus den humanen PBMCs durch Bilden von Komplexen aus humanen T-Zellen und magnetischen Kügelchen durch Mischen von mit Anti-CD3- und Anti-CD28-Antikörpern beschichteten magnetischen Kügelchen mit den humanen PBMCs;
    Schritt (3), Kultivieren der Komplexe aus humanen T-Zellen und magnetischen Kügelchen für 16 Stunden bis 20 Stunden, um eine T-Zellaktivierung zu erzielen, so dass Komplexe aus aktivierten humanen T-Zellen und magnetischen Kügelchen erhalten werden;
    Schritt (4), Addieren von CAR-exprimierendem Lentivirus zu den Komplexen aus aktivierten humanen T-Zellen und magnetischen Kügelchen für 40 Stunden bis 48 Stunden, um humane T-Zellen in CAR-T-Zellen auf den Komplexen aus humanen T-Zellen und magnetischen Kügelchen zu transduzieren, so dass Komplexe aus humanen CAR-T-Zellen und magnetischen Kügelchen erhalten werden;
    Schritt (5), Erhalten von humanen CAR-T-Zellen durch Dissoziieren der humanen CAR-T-Zellen von den Komplexen aus humanen CAR-T-Zellen und magnetischen Kügelchen; und
    Schritt (6), weiteres Kultivieren der humanen CAR-T-Zellen für 92 Stunden bis 96 Stunden, um die mit Tscm angereicherten, humanen CAR-T-Zellen zu erhalten.

2.  - Verfahren nach Anspruch 1, wobei die humanen PBMCs in Schritt (1) durch Isolieren aus humanem Vollblut oder Leukaphereseprodukt gesammelt werden.

3.  - Verfahren nach Anspruch 1 oder 2, wobei die Anzahl der mit Anti-CD3- und Anti-CD28-Antikörpern beschichteten magnetischen Kügelchen in Schritt (2) das 2-bis 4-Fache der Anzahl von humanen T-Zellen in den humanen PBMCs ist.

4.  - Verfahren nach einem der Ansprüche 1 bis 3, wobei die Komplexe aus humanen T-Zellen und magnetischen Kügelchen in Schritt (3) bei 36,5 °C bis 37,5 °C 16 Stunden bis 20 Stunden lang kultiviert werden.

5.  - Verfahren nach einem der Ansprüche 1 bis 4, wobei die Transduktion in Schritt (4) bei 36,5 °C bis 37,5 °C 40 Stunden bis 48 Stunden lang durchgeführt wird.

6.  - Verfahren nach einem der Ansprüche 1 bis 5, wobei die Transduktion in Schritt (4) eine Transduktion eines Gens, das Anti-CD19-, Anti-CD22-, Anti-B-Zellreifungsantigen-(BCMA-) oder Anti-Mesothelinrezeptor-Sequenzen umfasst, in humane T-Zellen ist.

7.  - Verfahren nach einem der Ansprüche 1 bis 6, wobei die humanen CAR-T-Zellen bei 36,5 °C bis 37,5 °C 92 Stunden bis 96 Stunden lang in Schritt (6) kultiviert werden.

8.  - Verfahren nach einem der Ansprüche 1 bis 7, wobei das Kultivieren der Komplexe aus humanen T-Zellen und magnetischen Kügelchen in Schritt (3), das Transduzieren von humanen T-Zellen in CAR-T-Zellen auf den Komplexen aus humanen T-Zellen und magnetischen Kügelchen in Schritt (4) und das weitere Kultivieren der humanen CAR-T-Zellen in Schritt (6) in komplettem X-VIVO™-15-Medium durchgeführt werden.

9.  - Verfahren nach einem der Ansprüche 1 bis 8, wobei die Anzahl der mit Tscm angereicherten, humanen CAR-T-Zellen, die in Schritt (6) erhalten werden, mehr als das 10-Fache der Anzahl von humanen T-Zellen ist, die unter Verwendung von magnetischen Kügelchen in Schritt (2) positiv selektiert werden.

10. - Verfahren nach einem der Ansprüche 1 bis 9, wobei die mit Tscm angereicherten, humanen CAR-T-Zellen, die in Schritt (6) erhalten werden, einen Prozentsatz an Tscm von mehr als 55 % aufweisen.

**Revendications**

1. - Procédé de production de lymphocytes T à récepteur antigénique chimérique (CAR-T) humains enrichis en lymphocytes T mémoire à cellules souches (Tscm), **caractérisé par le fait que** le procédé comprend :

   étape (1) la fourniture de cellules mononucléées du sang périphérique (PBMC) humaines ;
   étape (2) la sélection positive de lymphocytes T humains à partir des PBMC humaines en formant des complexes lymphocytes T humains-billes magnétiques, en mélangeant des billes magnétiques recouvertes d'anticorps anti-CD3 et anti-CD28 avec les PBMC humaines ;
   étape (3) la culture des complexes lymphocytes T humains-billes magnétiques pendant 16 heures à 20 heures pour l'activation des lymphocytes T afin d'obtenir des complexes lymphocytes T humains activés-billes magnétiques ;
   étape (4) l'ajout d'un lentivirus exprimant CAR aux complexes lymphocytes T humains activés-billes magnétiques pendant 40 heures à 48 heures, pour la transduction de lymphocytes T humains en lymphocytes CAR-T sur les complexes lymphocytes T humains-billes magnétiques pour obtenir des complexes lymphocytes CAR-T humains-billes magnétiques ;
   étape (5) l'obtention de lymphocytes CAR-T humains en dissociant les lymphocytes CAR-T humains des complexes lymphocytes CAR-T humains-billes magnétiques ; et
   étape (6) la culture supplémentaire des lymphocytes CAR-T humains pendant 92 heures à 96 heures pour obtenir les lymphocytes CAR-T humains enrichis en Tscm.

2. - Procédé selon la revendication 1, dans lequel les PBMC humaines à l'étape (1) sont collectées par isolement à partir de sang total humain ou d'un produit de leucaphérèse.

3. - Procédé selon l'une des revendications 1 ou 2, dans lequel le nombre de billes magnétiques recouvertes d'anticorps anti-CD3 et anti-CD28 à l'étape (2) est de 2 à 4 fois le nombre de lymphocytes T humains dans les PBMC humaines.

4. - Procédé selon l'une quelconque des revendications 1 à 3, dans lequel les complexes lymphocytes T humains-billes magnétiques à l'étape (3) sont cultivés de 36,5°C à 37,5°C pendant 16 heures à 20 heures.

5. - Procédé selon l'une quelconque des revendications 1 à 4, dans lequel la transduction à l'étape (4) est réalisée de 36,5°C à 37,5°C pendant 40 heures à 48 heures.

6. - Procédé selon l'une quelconque des revendications 1 à 5, dans lequel la transduction à l'étape (4) est la transduction d'un gène comprenant des séquences anti-CD19, anti-CD22, anti-antigène de maturation des lymphocytes B (BCMA) ou anti-récepteur de la mésothéline dans des lymphocytes T humains.

7. - Procédé selon l'une quelconque des revendications 1 à 6, dans lequel les lymphocytes CAR-T humains sont cultivés de 36,5°C à 37,5°C pendant 92 heures à 96 heures à l'étape (6).

8. - Procédé selon l'une quelconque des revendications 1 à 7, dans lequel la culture des complexes lymphocytes T humains-billes magnétiques à l'étape (3), la transduction des lymphocytes T humains en lymphocytes CAR-T sur les complexes lymphocytes T humains-billes magnétiques à l'étape (4), et la culture supplémentaire des lymphocytes CAR-T humains à l'étape (6) sont réalisées dans un milieu X-VIVO™ 15 complet.

9. - Procédé selon l'une quelconque des revendications 1 à 8, dans lequel le nombre de lymphocytes CAR-T humains enrichis en Tscm obtenus à l'étape (6) est supérieur à 10 fois le nombre de lymphocytes T humains sélectionnés positivement à l'aide de billes magnétiques à l'étape (2).

10. - Procédé selon l'une quelconque des revendications 1 à 9, dans lequel les lymphocytes CAR-T humains enrichis en Tscm, qui sont obtenus à partir de l'étape (6), présentent un pourcentage de Tscm supérieur à 55 %.

FIG.1

FIG.2

FIG.3

FIG.4A

EP 4 365 282 B1

FIG.4B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GATTINONI, LUCA et al.** A human memory T cell subset with stem cell-like properties. *Nature medicine*, 2011, vol. 17 (10), 1290-1297